# EUROPEAN PATENT APPLICATION

(11) **EP 4 603 592 A1**
(43) Date of publication of application: **20.08.2025**
(21) Application number: 23876785.9
(22) Date of filing: 12.10.2023
(51) Int. Cl.: C12Q 1/68

(54) **METHOD FOR GENERATING PROBES ON CARRIER AND METHOD FOR DETECTING TARGET SUBSTANCE IN SAMPLE**

(30) Priority: 14.10.2022 CN 202211261128; 09.10.2023 CN 202311305614
(71) Applicant: Shenzhen Salus Biomed Co., Ltd, Shenzhen, Guangdong 518107 (CN)
(72) Inventor: LIAO, Renjie, Shenzhen, Guangdong 518107 (CN); LIU, Erkai, Shenzhen, Guangdong 518107 (CN); WANG, Gufeng, Shenzhen, Guangdong 518107 (CN); CHEN, Shuang, Shenzhen, Guangdong 518107 (CN); ZHAO, Luyang, Shenzhen, Guangdong 518107 (CN)
(74) Representative: Cabinet Chaillot
(86) International application number: PCT/CN2023/124273
(87) International publication number: WO 2024/078585

(57) **Abstract**

The present invention discloses a method for generating probes on a carrier, and a method for detecting target substance in a sample. The method includes the following steps: hybridizing a probe template with oligonucleotides immobilized on a carrier, extending the oligonucleotides to obtain probes having different spatial barcode segments; performing first amplification to the probes to form probe clusters; sequencing the spatial barcode segments of the probes; performing re-amplification to the probe clusters; and forming capture segments for capturing the target substance on the probes. In this application, after sequencing to determine the spatial barcodes, re-amplification of the probe clusters is performed. The re-amplification by using the excess oligonucleotides on the carrier increases the number of the probes, such that the target substance in the sample can more readily "contact" the probes without relying on long-distance diffusion, which effectively addresses the problem of spatially uneven capture of target substance and enhances capture efficiency.

## Description

### CROSS-REFERENCE

The invention claims the priority of China Patent Application No. 202211261128.4, entitled "METHOD FOR GENERATING PROBES ON CARRIER AND METHOD FOR DETECTING TARGET SUBSTANCE IN SAMPLE", and filed on October 14, 2022 to China Patent Office, and also claims the priority of China Patent Application No. 2023113056146, entitled "METHOD FOR GENERATING PROBES ON CARRIER AND METHOD FOR DETECTING TARGET SUBSTANCE IN SAMPLE", and filed on October 09, 2023 to China Patent Office. The entire contents thereof are incorporated herein by reference.

### FIELD

The invention relates to the field of spatial omics, and more specifically, relates to a method for generating probes on a carrier and a method for detecting a target substance in a sample.

### BACKGROUND

The spatial omics technology provides important biological information support for cell classification, cell status characterization and cell-to-cell interaction through an in-situ characterization of tissue transcriptomes. Such technology is widely used in a plurality of biomedical fields such as neuroscience, research of tumor microenvironment, immunology, and disease marker discovery. The presently available spatial omics technology is mainly provided by 10x Genomics, whose spatial transcriptome product, Visium Spatial Gene Expression, enables spatial characterization of transcriptomes in FFPE (formalin fixed paraffin embedded) tissue sections and frozen tissue sections. However, such technology uses the method of DNA (deoxyribonucleic acid) microarray to construct the mRNA (message ribonucleic acid) capture chips, which has a relatively large limitation in resolution. Thus, there is a need to develop a spatial omics method with a higher capture efficiency.

### SUMMARY

An objective of the present application is to address at least one of the above technical problems. To this end, the present application proposes a method for generating probes on a carrier and a method for detecting a target substance in a sample. The probes generated on the carrier using this method can have a higher density and enhanced accessibility of mRNA, and enable more uniform and efficient mRNA capture.

According to one aspect of the present application, a method for generating probes on a carrier includes the following steps:
hybridizing a probe template with oligonucleotides immobilized on the carrier, extending the oligonucleotides to obtain probes having different spatial barcode segments;
performing first amplification to the probes to form probe clusters;
sequencing the spatial barcode segments of the probes;
performing amplification to the probe clusters; and
forming, on the probes, capture segments for capturing a target substance.

The method in accordance with this embodiment of the present application has at least the following advantages:
The applicant has discovered that, in existing spatial omics technologies, during the construction of capture probes, when spatial barcode segments are sequenced to determine the spatial barcodes corresponding to probe clusters at different locations on the carrier, in order to ensure accurate identification of spatial barcode segments across different probe clusters, inter-cluster gaps must be maintained which results in the probe clusters being discontinuously distributed on the carrier, thus leading to spatially uneven distribution of the origins of captured target substances. However, in this application, after sequencing to determine the spatial barcodes, re-amplification of the probe clusters is performed. The re-amplification by using the excess oligonucleotides on the carrier increases the number of the probes, such that the target substance in the sample can more readily "contact" the probes without relying on long-distance diffusion of the target substance, which effectively addresses the problem of spatially uneven capture of target substance and enhances capture efficiency.

In some embodiments of the present application, performing amplification to the probe clusters comprises performing re-amplification to the probe clusters such that the probe clusters having different spatial barcode segments are continuously distributed on the carrier.

In some embodiments of the present application, the capture segments are formed on the probes through ligase.

In some embodiments of the present application, the ligase includes T4 ligase.

In some embodiments of the present application, the capture segments each comprise a plurality of continuous T bases.

In some embodiments of the present application, the number of the continuous T bases is 10-50.

In some embodiments of the present application, at least one of the T bases is modified with locked nucleic acid.

In some embodiments of the present application, at least one, two, three, four or more of the T bases are modified with locked nucleic acid.

In accordance with a second aspect of the present application, a carrier is provided, which is formed with probes thereon by adopting the above-mentioned method.

In some embodiments of the present application, the carrier is a solid-phase carrier.

In some embodiments of the present application, the solid-phase carrier includes a flow cell.

In accordance with a third aspect of the present application, a method for detecting a target substance in a sample is provided, including the following steps:
generating probes on a carrier in accordance with the above method or providing the above carrier; and
making the sample contact the carrier to enable the probes to specifically bind to the target substance through the capture segments.

In some embodiments of the present application, the method further includes:
extending the probes to obtain nucleic acid molecules containing the spatial barcode segments and the target substance; and
determining the location of the target substance in accordance with the spatial barcode segments of the nucleic acid molecules.

In some embodiments of the present application, the target substance is mRNA or oligonucleotide.

In some embodiments of the present application, extending the probes to obtain nucleic acid molecules containing the spatial barcode segments and the target substance comprises: extending the probes by reverse transcription to obtain nucleic acid molecules containing the spatial barcode segments and reverse-transcribed sequence of the target substance.

In some embodiments of the present application, determining the location of the target substance in accordance with the spatial barcode segments of the nucleic acid molecules comprises: separating the nucleic acid molecules from the carrier, sequencing the nucleic acid molecules, and determining the location of the target substance in accordance with the sequence of the spatial barcode segments.

In some embodiments of the present application, separating the nucleic acid molecules from the carrier may include separating both strands of double-stranded nucleic acid molecules from the carrier, or separating synthetic strand of double-stranded nucleic acid molecules from the carrier. Thus, the sequence of the spatial barcode segment can be the corresponding sequence itself or the complementary sequence of the synthesized strand.

In some embodiments of the present application, the sample is a tissue section.

In some embodiments of the present application, making the sample contact the carrier to enable the probes to specifically bind to the target substance through the capture segments comprises: making the sample contact the carrier, and permeabilizing the sample, to enable the probes to specifically bind to the target substance in the sample through the capture segments.

### BRIEF DESCRIPTION OF THE DRAWINGS

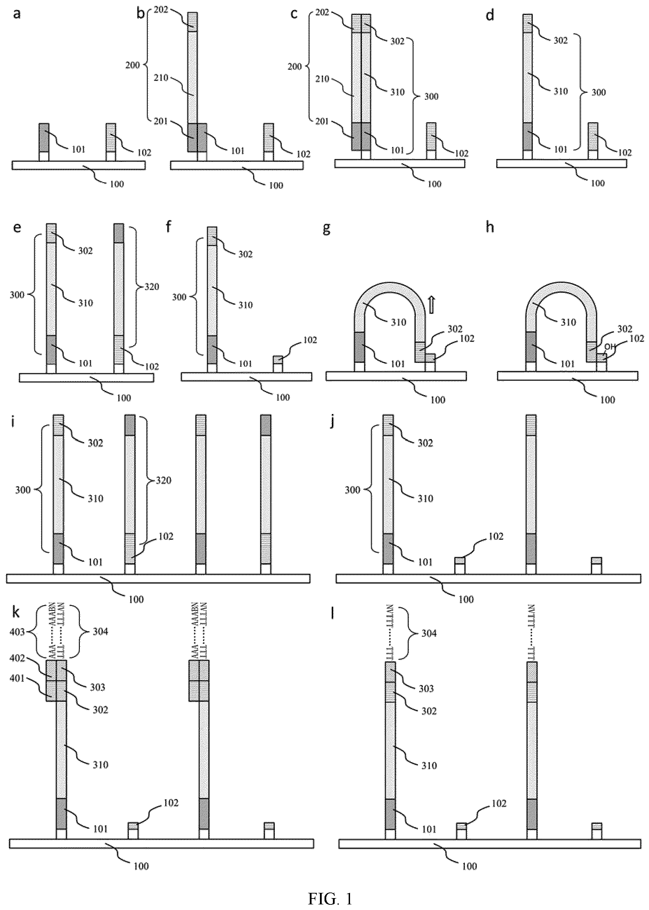

FIG. 1 is a schematic diagram showing a method for generating probes on a carrier in accordance with an embodiment of the present application, wherein the part "a" shows that oligonucleotides are immobilized on a carrier, the part "b" shows that a probe template is hybridized with the oligonucleotides, the part "c" shows that the oligonucleotide is extended to obtain a probe, the part "d" shows denaturation and removal of the probe template, the part "e" shows first amplification, the part "f" shows linearization, the part "g" shows sequencing, the part "h" shows dephosphorylation and washing away of the sequencing strand, the part "i" shows re-amplification, the part "j" shows linearization after the re-amplification, the part "k" shows that capture segments are ligated to primers through ligase by using guide segments, and the part "l" shows denaturation and removal of the guide segments.
FIG. 2 is a schematic diagram showing a comparison between the method without reamplification and the method with re-amplification in accordance with the present application;
FIG. 3 is a schematic view showing a structure of the probe wherein a capture segment has not been formed, in accordance with the present application;
FIG. 4 shows that mRNA is captured by the probe and subjected to sequencing in accordance with the method for detecting target substance of the present application, wherein the part "a" shows that the target substance is captured and the target substance cDNA is formed on the probe through reverse transcription, the part "b" shows synthesis of synthetic strand using the probe containing target substance cDNA as a template, and the part "c" shows library preparation and sequencing after denaturation and elution of the synthetic strand.
FIG. 5 shows a spatial distribution heatmap of captured mRNA in accordance with an embodiment 2 of the present application.
FIG. 6 shows a spatial distribution heatmap of captured gene counts in accordance with the embodiment 2 of the present application.
FIG. 7 shows a spatial distribution heatmap of captured mRNA in accordance with an embodiment 3 of the present application.
FIG. 8 shows a spatial distribution heatmap of captured gene counts in accordance with the embodiment 3 of the present application.
FIG. 9 shows a spatial distribution heatmap of captured mRNA in accordance with an embodiment 4 of the present application.
FIG. 10 shows a spatial distribution heatmap of captured mRNA in accordance with a comparative example 1.
FIG. 11 shows a spatial distribution heatmap of captured molecule counts of a mouse brain tissue using the Visium slide.
FIG. 12 shows a spatial distribution heatmap of captured molecule counts of a mouse brain tissue using the chip in accordance with an embodiment of the present application.

Reference numerals: 100: carrier, 101: first adapter, 102: second adapter, 200: probe template, 201: first adapter template, 202: second complementary adapter template, 210: intermediate template, 300: first probe, 302: second complementary adapter, 303: linker sequence, 304: capture sequence, 305: target substance cDNA segment, 310: first probe intermediate part; 311: first adapter sequencing primer, 312: spatial barcode segment, 313: spatial barcode segment sequencing primer, 320: second probe, 401: second adapter corresponding sequence, 402: linker corresponding sequence, 403: capture corresponding sequence, 500: target substance, 600: synthetic strand, 701: first library primer, 702: second library primer.

### DESCRIPTION OF THE EMBODIMENTS

For facilitating full understanding of the purposes, technical features, and effects of the present invention, a clear and complete description of the concept and technical effects of the present application is provided below with reference to embodiments. Obviously, it is to be understood that the specific embodiments as disclosed are only a part of the embodiments, rather than all embodiments of the present application. Based on the disclosed embodiments of the present application, other embodiments that those skilled in the art can obtain without spending creative effort shall fall within the protection scope of the present application.

In the following detailed description of the embodiments of the present application, the disclosed embodiments are illustrative, only for explaining the present application, which cannot be regarded as limiting the present invention.

In the description of the present application, the term "at least one" means one or more. The term "plurality" means two or more. The term "Greater than", "less than", or "exceeding" means that the number specified is exclusive, and "above", "under" or "within" means that the number specified is inclusive. If "first", "second" are mentioned, they are used for the purpose of distinguishing the technical features, rather than being understood to indicate or hint the relative importance, or hint the indication of the amount of the listed technical features, or hint the indication of the sequence relation of the listed technical features. In the following description, although the flow chart shows the logic sequence, under certain circumstances, the shown or described steps can be performed in a sequence different from that of the flow chart.

Unless otherwise defined, all technical and scientific terms used herein shall have the same meanings as commonly understood by those skilled in the technical field of this application. The terms used in this specification are intended solely to describe specific embodiments and not to limit the application.

In the description of this application, references to terms such as "one embodiment," "some embodiments," "illustrative embodiments," "example," "specific examples," or "some examples" mean that the specific features, structures, materials, or characteristics described in connection with the embodiment or example are included in at least one embodiment or example of this application. In this specification, schematic descriptions of the aforementioned terms do not necessarily refer to the same embodiment or example. Furthermore, the described specific features, structures, materials, or characteristics may be combined in any suitable manner in one or more embodiments or examples.

The present application proposes a method for generating probes on a carrier, wherein the carrier includes a rigid part which can immobilize oligonucleotides thereon. A material for the rigid part can be chosen from at least one of PMMA (poly(methyl methacrylate)), PDMS (polydimethylsiloxane), PET (polyethylene terephthalate), PC (polycarbonate), PS (polystyrene), epoxy resin, glass, silicon sheet, etc. The number of the nucleotides in the oligonucleotides ranges from 2 to 100, optionally between 2 to 50, or even further optionally between 2 to 30. For example, the number can be 2, 3, 4, 5, 8, 10, 15, 20, 25 or 30. The immobilization of the oligonucleotides on the rigid part is realized in a manner including, but not limited to, at least one of physical adsorption (for example, through non-covalent interaction including at least one of polar interaction, hydrophobic interaction, or electrostatic interaction), covalent coupling (for example, amino-carboxyl reaction, using EDC (1-Ethyl-3-(3-dimethylaminopropyl) carbodiimide hydrochloride), CMC (carboxymethyl cellulose), APTES (3-aminopropyl)triethoxysilane), glutaraldehyde as a coupling agent), Thiol-based coupling, click chemistry reaction, Glycosyl immobilization (for example, sodium periodate oxidation, or sodium borohydride-mediated conjugation), staudinger reaction, Diels-Alder reaction, native chemical ligation, affinity interaction (for example, avidin-biotin).

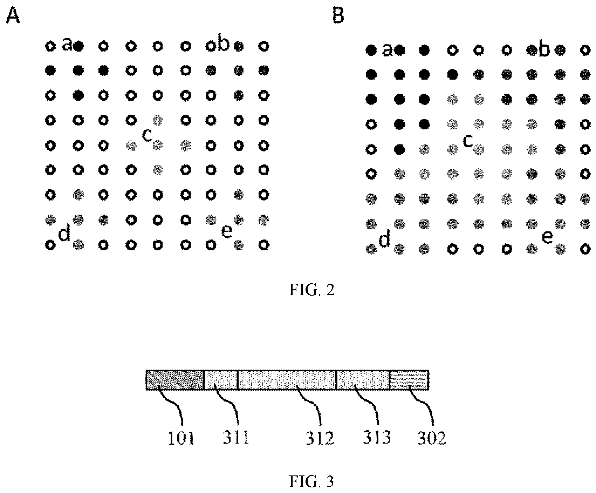

Referring to FIG. 1, in the method of generating probes on a carrier according to the present application, the carrier 100 is provided with oligonucleotides 101 pre-immobilized thereon. A probe template 200 is hybridized with the oligonucleotides 101. The oligonucleotides 101 are extended to obtain first probes 300. Referring to FIG. 3, the first probe 300 includes a spatial barcode segment 312. The spatial barcode segment 312 is a nucleotide fragment on the first probe 300 for indicating a location of the first probe 300 on the carrier 100, which can be continuous or discontinuous and typically has a length of six or more nucleotides. In some specific embodiments, the length of the spatial barcode segment 312 is 6-30 nucleotides, optionally 6-25 nucleotides, or even further optionally 6-20 nucleotides; for example, it can be 6, 7, 8, 9, 10, 11, 12, 13, 14, 15, 16, 17, 18, 19 or 20 nucleotides. The first probes 300 at different locations on the carrier 100 have different spatial barcode segments 312. The first probes 300 having the same spatial barcode segment 312 are deemed as being positioned at the same location on the carrier 100 to distinguish different locations on the carrier 100, whereby the resolution of the first probes 300 is established in accordance with the distance between the first probes 300 at different locations.

After the oligonucleotides 101 are extended to form the first probes 300, amplification is performed to form probe clusters on the carrier 100. At this moment, corresponding locations of the different probes 300 on the carrier 100 need to be determined. Thus, the spatial barcode segments 312 of the first probes 300 are sequenced. Since the first probes 300 at different locations on the carrier 100 have different spatial barcode segments 312, sequencing probes at specific locations allows for identification of sequences of the spatial barcode segments at those specific locations, thereby associating the locations of the respective probe clusters with their specific sequences of the spatial barcode segments 312 of the probe clusters.

The applicant has discovered that, in this process, in order to ensure accurate identification of spatial barcode segments across different probe clusters, inter-cluster gaps must be maintained which results in the probe clusters being discontinuously distributed on the carrier. To address this, in this application, after sequencing of the spatial barcode segments is finished, re-amplification is performed to increase the number of the probes within the same region, such that in subsequent detection, the target substance in the sample can more readily "contact" the probes without relying on long-distance diffusion of the target substance on the carrier.

Referring to FIG. 2, it is a schematic diagram illustrating the difference between the principles of the method in accordance with the embodiment of the present application and the existing method. The part "a" shows that the original carrier is formed with five probe clusters a-e thereon through a single amplification. The single amplification for forming the probe clusters amplifies probe signals during spatial barcode sequencing to distinguish them from the background fluorescence. To ensure that the locations of the five probe clusters can be correctly identified during sequencing, the five probe clusters are spaced apart from each other. This arrangement leaves many oligonucleotide sites on the carrier unoccupied by probes. The target substance in the sample within these regions may fail to bind to probes, thus forming blank regions, or require a longer diffusion distance to be captured by the probes, thus leading to spatial unevenness and relatively low capture efficiency in target substance capture. In contrast, the part "b" shows the five probe clusters a-e on the carrier that are obtained through re-amplification in accordance with the embodiment of the present application. Through the re-amplification, the number of the probes in each probe cluster is increased, and the target substance in the sample can more easily contact the probes so as to be more easily captured, which greatly improves the spatial distribution uniformness as well as capture efficiency.

In some specific embodiments, the re-amplification ensures that the probe clusters with different spatial barcode segments are continuously distributed on the carrier. Here, "continuous distribution" typically refers to a situation where, between adjacent probe clusters with different spatial barcode segments, only a few of oligonucleotide sites for probe formation remain unoccupied with probes. For example, at most 1/5, 1/6, 1/7, 1/8, 1/9, 1/10, 1/12, 1/15, 1/20, 1/30, 1/40, 1/50, 1/100, 1/150, 1/200 of the sites are unoccupied with probes, while other sites are formed with probes during the processes of the hybridization with the probe template, the amplification or the re-amplification. Further optimal situation is that all oligonucleotide sites for probe formation between adjacent probe clusters with different spatial barcode segments are fully occupied with probes, with adjacent probe clusters being contiguous. This maximizes the number of the probes on the carrier, which enables the target substances in the sample to be captured as much as possible, thereby further enhancing the target substance capture efficiency and spatial resolution.

After the re-amplification is completed, the probes are formed with capture segments for capturing the target substance, wherein the capture segments refer to nucleotide fragments which can specifically bind to the target substance. With specific designs of the nucleotide fragments of the capture segments, specific binding to the target substances can be realized.

In some specific embodiments, the capture segments are formed by nucleotide fragments linked to the probes via the action of ligase. Compared with enzymatic digestion to expose the capture segments from the original probes, employing a ligase to ligate it to the probes overcomes the limitations of enzymatic digestion efficiency. For probes that have not undergone successful enzymatic digestion, although the target substance can still be captured through the specific binding of the capture segments, the captured target substance is unable to successfully undergo reverse transcription to form nucleic acid molecules containing the spatial barcode segments and the target substance, thus losing information of this part of target substance. Consequently, the final obtained information about the target substances and their spatial distribution becomes incomplete. However, for some probes whose capture segments are linked via a ligase, if the capture segments fail to ligate, the target substance, while unable to be captured by these probes, can diffuse to adjacent probes for capture, without directly causing loss of target substance, thereby significantly reducing the possibility of information loss. The ligase may be any ligase capable of ligating a capture segment to a probe, such as a ligase that utilizes ATP or NAD to catalyse the ligation of the nucleotide fragment to the probe to form the capture segment. The sources of the ligase include, but are not limited to, prokaryotes, eukaryotes, viruses, etc., and may include a natural ligase or a ligase having the same function that is obtained by substitution, deletion, and/or addition of one or more amino acid residues. Specific examples include, but are not limited to, T4 DNA ligase, T7 DNA ligase, Taq DNA ligase, E. coli DNA ligase.

The target substance to be captured by the capture segment can be any biological macromolecule, small molecule, ion, organism, etc., such as at least one of proteins, peptides, nucleic acids, nucleotides, polysaccharides, lipids, lipopolysaccharides, lipoproteins, glycoproteins, steroids, anions, cations, viruses, bacteria, or cells. In some specific embodiments, the target substance is a nucleic acid fragment, specifically an RNA fragment or DNA fragment, and more specifically mRNA. Since mRNA typically contains a polyA tail, specific binding to mRNA can be realized by designing multiple consecutive T bases (thymine bases) in the capture segment. It will be understood that the target substance may also be an oligonucleotide, which can serve as a tag for being linked with other small or macromolecules, such as forming antibody-oligonucleotide conjugates by conjugation to an antibody, or serve as a tag for being linked in another manner. In some embodiments, different oligonucleotide, as tags, are respectively conjugated to different antibodies, enabling corresponding antibody information to be obtained through the oligonucleotide tags. In some specific embodiments, the number of consecutive T bases in the capture segment ranges from 10 to 50, and optionally 10-40, 10-30, or 20-30. In some specific embodiments, at least one locked nucleic acid (LNA)-modified thymine is introduced into the consecutive T bases to enhance the affinity of the capture segment for capturing mRNA. Further, at least two LNA-modified T bases, or at least three LNA-modified T bases, may be introduced into the consecutive T bases to significantly improve the affinity of the capture segment for mRNA capture.

In some specific embodiments, referring to Fig. 1, the oligonucleotides immobilized on the carrier 100 include first adapters 101 capable of hybridizing with the probe template 200, and second adapters 102. In some embodiments, the second adapter 102 further includes at least one site capable of inducing breakage, such as a specific enzymatic cleavage site, which may specifically be modified nucleotides (e.g., i8oxoG or isoxyU) that enable enzymatic cleavage under the action of specific enzyme, thereby linearizing the probe. In some specific embodiments, the second adapter 102 includes two cleavage sites capable of inducing breakage, allowing for respective linearization under the action of different enzymes. Thus, after the first amplification, cleavage of one strand of the double-stranded amplification product can be achieved using one cleavage site, and after re-amplification, cleavage of one strand of the double-stranded amplification product can be achieved using the other cleavage site.

In some specific embodiments, the first amplification is a bridge amplification. The probe template 200 includes a first adapter template 201 and a second complementary adapter template 202. The first adapter template 201 and the second complementary adapter template 202 respectively contain sequences complementary to the first adapters 101 and the second complementary adapters 302. The bridge amplification of the probes is realized by utilizing the first adapters 101 and the second complementary adapters 302, thereby obtaining the first probes 300 and second probes 320. In some embodiments, there is an intermediate template 210 between the first adapter template 201 and the second complementary adapter template 202. The intermediate template 210 includes a template sequence corresponding to the spatial barcode segment.

In some specific embodiments, referring to FIG. 1, after the bridge amplification, through linearization, the second adapter 102 on the second probe 320 is washed away, while the first probe 300 formed on the first adapter 101 is reserved, and the spatial barcode segment in the first probe 300 is then subjected to sequencing. In some embodiments, the method for sequencing the spatial barcode segment includes Sanger sequencing, NGS (next-generation sequencing) sequencing, nanopore sequencing, etc. Preferably, it adopts the NGS sequencing. In some embodiments, after the sequencing, the sequencing strand is washed away and the re-amplification is performed using the remaining oligonucleotides on the carrier 100. After the re-amplification is completed, similar linearization is performed to wash away the second probe 320 which is newly produced by the re-amplification on the second adapter 102, while keeping the first probe 300 on the first adapter 101. As a result, most of the first adapters are formed with the first probes 300. The first adapters 101 then act as oligonucleotide sites suitable for probe formation. Only a small number of the sites are not formed with probes, while the other sites of the first adapters are all formed with the probes during the hybridization with the probe template, the first amplification or the re-amplification process.

In some specific embodiments, the capture segment is formed on the probe through a guide segment and a ligase, wherein the guide segment includes a second adapter corresponding sequence 401which is at least partially complementary to the second complementary adapter 302 (for example, it can be complementary to 15-20 bases in the second complementary adapter 302), and a linker corresponding sequence 402. In some embodiments, through an additional capture corresponding sequence 403 of the guide segment which is adjacent to the linker corresponding sequence 402, a capture segment including a capture sequence 304 is finally formed on the probe. In other preferred embodiments, for reducing mismatches, the guide segment contains only the second adapter complementary sequence 401 and the linker corresponding sequence 402. The guide segment is hybridized to the probe through complementary pairing between the second adapter complementary sequence 401 and the second complementary adapter 302. Meanwhile, the capture segment includes a linker sequence 303 and a capture sequence 304, and is hybridized to the guide segment through the complementary pairing between the linker sequence 303 and the linker corresponding sequence 402 of the guide segment. In this way, after the probe and the guide segment are hybridized together, and the guide segment and the capture segment are hybridized together, the capture sequence 304 is introduced into the probe via the ligase through ligation mediated by the linker sequence 303 and the second complementary adapter 302. In some embodiments, the capture sequence 304 includes a plurality of consecutive T bases, for example, T30VN. In some embodiments, after the introduction of the capture segment onto the probe, the method further includes washing away the guide segment to form a capture probe carrying the capture segment.

Thus, in some of the above-mentioned specific embodiments, the first probe includes a first adapter, a first probe intermediate portion, and a second complementary adapter. The first probe intermediate portion includes a first adapter sequencing primer, a spatial barcode segment, and a spatial barcode segment sequencing primer. The first adapter is complementary to the first adapter template of the probe template. The first adapter intermediate portion is complementary to the intermediate template of the probe template. The second complementary adapter is complementary to the second complementary adapter template of the probe template, and meanwhile the second complementary adapter is the same as the second adapter, wherein "complementary" and/or "same" means that the sequences have at least 50% of their bases which are the same and/or complementary at their corresponding positions. Optionally, they have 60%, 70%, 80%, 85%, 86%, 87%, 88%, 89%, 90%, 91%, 92%, 93%, 94%, 95%, 96%, 97%, 98%, 99% of their bases which are complementary and/or the same at their corresponding positions.

In some specific embodiments, referring to FIG. 3, the probe thus formed includes the first adapter 101, the first adapter sequencing primer 311, the spatial barcode segment 312, the spatial barcode segment sequencing primer 313, the second complementary adapter 302, the linker sequence 303, and the capture sequence 304. The spatial barcode segment sequencing primer 313 is used as a corresponding primer for sequencing the spatial barcode segment 312. The first adapter sequencing primer 311 is used as a corresponding primer for sequencing the full-length sequence formed after the capture.

In some specific embodiments, referring to FIG. 1 in combination with FIG. 2, the method for generating probes on a carrier includes the following steps:
hybridizing a probe template 200 with oligonucleotides immobilized on the carrier 100 that include first adapters 101, and extending the oligonucleotides to obtain first probes 300 which include different spatial barcode segments 312 (a-d of FIG. 1);
performing first amplification to the probes in the form of bridge amplification to form probe clusters including the first probes 300 and second probes 320 respectively formed on the first adapters 101 and the second adapters 102 (e of FIG. 1);
removing the second probes 320 on the second adapters 102, and sequencing the spatial barcode segments 312 of the first probes 300 on the first adapters 101 (f-h of FIG. 1);
performing re-amplification to the probe clusters, and removing the second probes on the second adapters 102 again (i and j of FIG. 1); and
ligating capture segments for capturing target substances to the first probes through a ligase to obtain capture probes (k and j of FIG. 1).

Embodiments of the present application further provide a carrier having probes formed thereon through the method mentioned above. The probes on the carrier formed by the present method have significantly reduced spacings therebetween, whereby the spatial uniformity during the capturing process can be improved. The density of the probes, the accessibility of the target substance to the probes, and the capture efficiency can also be enhanced.

In some specific embodiments, the carrier is a solid-phase carrier and may specifically include a flow cell.

Embodiments of the present application further provide a method for detecting a target substance in a sample. The method includes generating probes on a carrier using the above-mentioned method. After the formation of the probes, the sample is made to contact the carrier. The probes specifically bind to the target substance through the capture segments of the probes.

To achieve in-situ characterization of the sample, the spatial omics needs to localize the target substance to be detected to determine its origin within the sample. In the present application, through the above-mentioned detection method, the target substance is added with the spatial barcode segment capable of tagging the positional origin of the target substance, thereby achieving the localization function. Since the density of the capture probes on the carrier and accessibility of the target substance are significantly enhanced, the capture efficiency is thus improved.

In some specific embodiments, the sample can be a tissue sample, for example, a sample of a tissue section. Specifically, the sample can be a tissue sample derived from animal cells or plant cells. For example, the tissue sample may originate from organs such as the motion system, nervous system, endocrine system, circulatory system, respiratory system, digestive system, urinary system, reproductive system, etc. The tissue of the tissue sample can be an in vivo tissue or an ex vivo cultured viable tissue. It should be understood that the tissue can also be an organoid formed from one or more types of tissues or cells, for example, an organoid derived from a normal tissue or a tumor tissue, or an organoid derived from induced pluripotent stem cell, etc. In the meantime, the type of the organoid is, for example, at least one of the following: eye organoids, brain organoids, heart organoids, lung organoids, liver organoids, pancreatic organoids, kidney organoids, epithelial organoids, etc.

For facilitating probe-mediated capture of the target substance, the target substance in the sample is released through a permeabilization process. Specifically, the process includes bringing the sample into contact the carrier, and permeabilizing the sample to enable the probes to specifically bind to the target substance in the sample through the capture segments. The process for permeabilizing the sample can include chemical treatment, thermal treatment, ultrasonication, enzymatic treatment, hypotonic treatment, etc. for releasing the target substance from the sample.

In some specific embodiments, the substance is nucleic acid, or specifically mRNA. The mRNA can thus be reverse-transcribed to cDNA through probe extension to obtain a nucleic acid molecule containing the spatial barcode segment and the target substance cDNA. Localization of the target substance is achieved according to the spatial barcode segment of the nucleic acid molecule.

In some specific embodiments, localization of the target substance according to the spatial barcode segment of the nucleic acid molecule includes: separating the nucleic acid molecule from the carrier, sequencing the nucleic acid molecule, and determining the location of the target substance according to the sequence of the spatial barcode segment.

In some specific embodiments, the method for detecting the target substance in the sample includes the following steps:
generating probes on a carrier according to the above-mentioned method;
proving a sample, attaching the sample to the carrier, and immobilizing the sample on the carrier;
permeabilizing the sample to release the target substance mRNA from the sample, and making the probes specifically bind to the target substance mRNA through the capture segments;
extending the probes via reverse transcription to synthesize a cDNA segment corresponding to the target substance mRNA, thereby obtaining a probe chain containing the spatial barcode segments and a cDNA segment of the target substance;
digesting the tissue;
washing away the captured target substance mRNA, synthesizing a synthetic strand complementary to the probe chain using the probe chain as a template, then denaturizing, eluting, and sequencing the synthetic strand, and localizing the origin of the target substance mRNA in the sample according to the corresponding sequence of the spatial barcode segment.

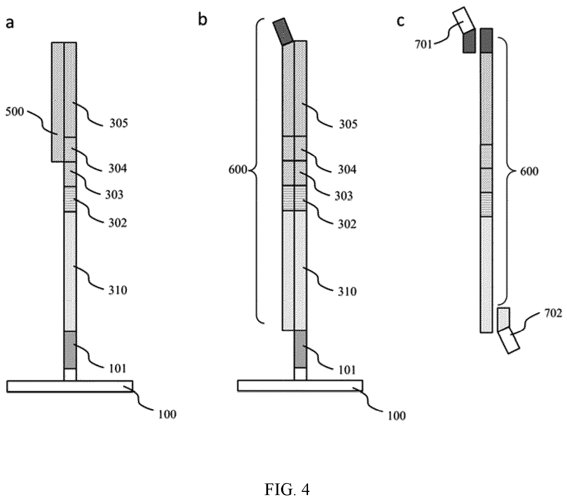

Referring to FIG. 4 in combination with FIG. 1, after the first probes are generated on the carrier 100 and capture the target substance 500, a target substance cDNA segment 305 is formed on a probe chain through reverse transcription. The target substance 500 is thereafter removed by denaturization. The probe chain of the first probe is used as a template to synthesize a synthetic strand 600, which is denaturized again to obtain a free synthetic strand 600. Library preparation and sequencing are performed using a first sequencing primer 701 and a second sequencing primer 702 to obtain the sequences of the target substance and its corresponding spatial barcode segment. The corresponding location of the target substance on the carrier 100 is obtained according to the sequence corresponding to the spatial barcode segment, thereby achieving the localization of the target substance.

### EMBODIMENT 1

This embodiment relates to a microfluidic chip. P7 (5'DBCO/iSp18//iSp18/TTTTTTTTTTCAAGCAGAAGACGGCATACGAGAT, SEQ ID No. 1) and P9 (5'DBCO/iSp18//iSp18/TTTTTTTTTTAAT/i8oxoG/ATACGGCGACCACCGAGA/ideoxyU/C TACAC, SEQ ID No. 2) are immobilized on the chip as the first adapter and the second adapter, respectively. Two nucleotides, i.e., i8oxoG and ideoxyU, are incorporated into P9, respectively serving as enzyme cleavage sites for a USER (Uracil-Specific Excision Reagent) enzyme and a fpg (Formamidopyrimidine Glycosylase) enzyme. Here, a redesigned P9 sequence is adopted to avoid conflicts with P5 in the mRNA capture library during subsequent library preparation and sequencing.

This embodiment further relates to a probe template library, where the probe includes a sequencing adapter sequence 1, a sequencing primer sequence, a spatial barcode segment sequence, a spatial barcode segment sequencing primer sequence, and a sequencing adapter sequence 2.

The sequencing adapter sequence 1 is reversely complementary to the P7 sequence. The sequencing adapter sequence 2 is reversely complementary to the P9 sequence. The sequencing primer sequence can be obtained by referring to specific sequencing platforms or can be self-designed. The spatial barcode segment sequence consists of 20-30 random bases, enabling the generation of about 10⁸ or an even larger amount of spatial codes for positional differentiation. The spatial barcode segment sequencing primer sequence can be self-designed according to the spatial barcode segment sequence.

This embodiment further provides a method for generating probes on a carrier. Referring to the standard paired-end sequencing process, the method includes the following steps:

### (1) Capture probe seed library hybridization and bridge amplification:

A probe template library is hybridized on the chip and extension is performed to obtain the probes. Bridge amplification is performed to form a plurality of probe clusters on the chip, with each probe cluster containing a plurality of probes having the same sequence, and each probe cluster having a unique spatial barcode segment.

### (2) sequencing the spatial barcode segment:

The primers are hybridized, the spatial barcode segment is sequenced, and spatial positioning for each spatial barcode segment is performed according to an image to thereby obtain spatial coordinates of each spatial barcode.

### (3) bridge re-amplification and linearization:

The sequenced DNA strand of the spatial barcode is washed away by using a denaturing reagent, followed by cleaning using a cleaning reagent. After using PNK (Polynucleotide Kinase) enzyme to conduct terminal dephosphorylation, a second bridge amplification is performed to populate the chip with mRNA capture probes, which form continuous probe clusters contiguous to each other. Thereafter, the fpg azyme is used to conduct the linearization, leaving only the DNA strand of the sequencing adapter 1 on the chip.

### (4) mRNA capture sequence ligation

The mRNA capture probe (including a linker sequence and a capture sequence T30VN) is ligated to the probe using T4 ligase through a guide segment including a sequencing adapter 2 complementary sequence, a linker corresponding sequence, and a capture corresponding sequence (A30BN). The reaction system is as follows: 2 U/µL T4 ligase, 1 µM guide segment (the second adapter corresponding sequence 401 and linker corresponding sequence 402 of FIG 1), 1µM mRNA capture probe (the linker sequence 303 and the capture sequence 304), 1X ligase buffer, 0.5% PEG 2000 (Polyethylene glycol 2000), incubate at 37 °C, and reaction overnight. Then, the guide segment is washed away by using a denaturing reagent and cleaned three times by using a cleaning reagent.

### EMBODIMENT 2

This embodiment provides a method for detecting a target nucleic acid. The method includes the following steps:

### (1) Tissue sectioning and attachment:

A mouse liver tissue is cut into 10 µm thick sections by using a cryostat, which are attached to a chip having the mRNA capture probes prepared in EMBODIMENT 1.

### (2) Tissue fixation:

Methanol is pre-cooled to -20 °C. The chip with the tissue is fixed in the iced methanol for 30 minutes and then washed with ultrapure water three times.

### (3) Tissue permeabilization to release mRNA:

A permeabilizing processing is conducted to the tissue by using collagenase (collagenase I, 0.2 U/µL, in HBSS (Hank's Balanced Salt Solution) buffer, 37 °C, 20 minutes). The tissue is washed by using 0.1×SSC (Saline-Sodium Citrate). Then it is processed (37 °C, 10 minutes) by using pepsin (1 mg/mL pepsin dissolved in 0.1 M HCl (hydrochloric acid)). Finally, it is washed by using 0.1×SSC.

### (4) Reverse transcription

A reverse transcription mixture reagent is added, which contains:
2 µL Maxima H-RTase,
1 µL RNase inhibitor,
8 µL 5X Maxima 5x RT Buffer,
4 µL 10 mM dNTP mix,
8 µL 20% Ficoll PM-400,
4 mL Actinomycin D, and
13 µL Nuclease free water;

They are incubated at 42 °Covernight. After the reaction, the reaction solution is discarded, and the Exonuclease I (1U Exol in 50 µL 1×ExoI buffer) is added to react at 37 °C for 45 minutes.

### (5) Tissue digestion:

The tissue is incubated in 1×digestion buffer for digestion at 37 °C for 45 minutes. Then it is washed three times by using 0.1×SSC.

### (6) Washing away the mRNA strand:

The mRNA strand is washed away by using 0.1 M NaOH (three times, each time lasting for 5 minutes). Then, 0.1 M Tris (pH=7.5) is used for neutralization, and ultrapure water is used for washing for three times.

### Synthesizing the synthetic strand complementary to cDNA:

The capture probe containing the cDNA is used as the template strand. The template strand is added with a second strand synthesizing mixture (3µL Klenow Fragment, 3 µL NEB buffer, 3µL 10 mM dNTP mix, 3 µL 100 µM primer with 9 random nucleotide sequences (TCAGACGTGTGCTCTTCCGATCTNNNNNNNNN, SEQ ID No. 3), 18 µL Nuclease-free water). Reaction takes place at 37 °C for 2 hours to synthesize the synthetic strand complementary to the template strand. Then, the tissue is washed three times by using the ultrapure water.

### (8) Washing away the synthetic strand:

The synthetic strand is washed away from the chip by using 0.1 M NaOH. Then it is neutralized by using 0.1 M Tris (pH=7.5).

### (9) Library preparation and sequencing:

The synthetic strand is purified by using 1.8×AMPure XP magnetic beads. Then PCR amplification is performed by using Kapa Hifi Hotstart Readymix, wherein the system includes the following primers:
TCTTTCCCTACACGACGCTC (SEQ ID No. 4), and
TCAGACGTGTGCTCTTCCGA (SEQ ID No. 5), having a concentration of 2 µM.

PCR conditions: 95 °C for 3 minutes, 15 cycles of (90 °C for 30 seconds, 60 °C for 1 minute, and 72 °C for 1 minute), 72 °C for 2 minutes, final hold at 4 °C. The product of the PCR is purified by using 1.2×AMPure XP magnetic beads.

A second amplification is performed to the product by using Kapa Hifi Hotstart Readymix, wherein the system includes the following primers:
AATGATACGGCGACCACCGAGATCTACACTCTTTCCCTACACGACGCTCTTC (SEQ ID No. 6), and
CAAGCAGAAGACGGCATACGAGATGTGACTGGAGTTCAGACGTGTGCTCTT CCGA (SEQ ID No.7), each having a concentration of 1 µM.

PCR conditions: 95 °C for 3 minutes, 8 cycles of (90 °C for 30 seconds, 60 °C for 30 seconds, and 72 °C for 30 seconds), 72 °C for 2 minutes, final hold at 4 °C. The product of the PCR is purified by using 0.8×AMPure XP magnetic beads. Finally, sequencing is performed to the final library (paired-end sequencing, PE100-150).

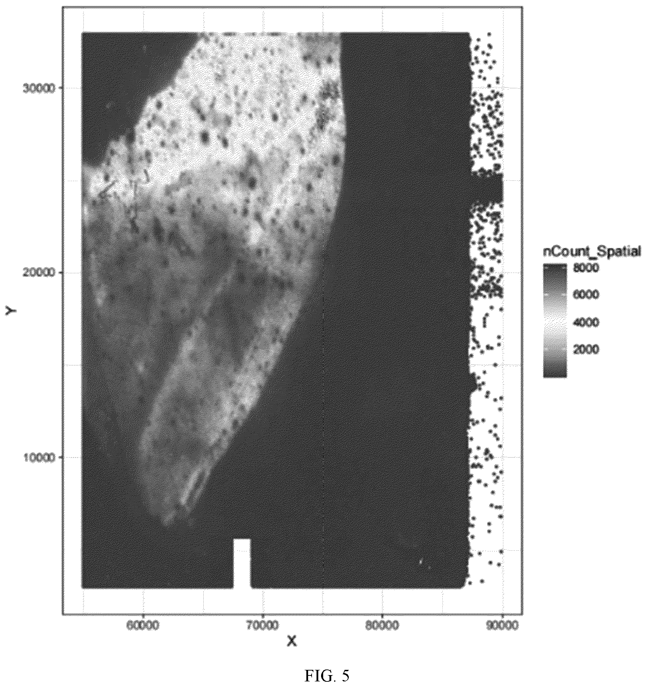

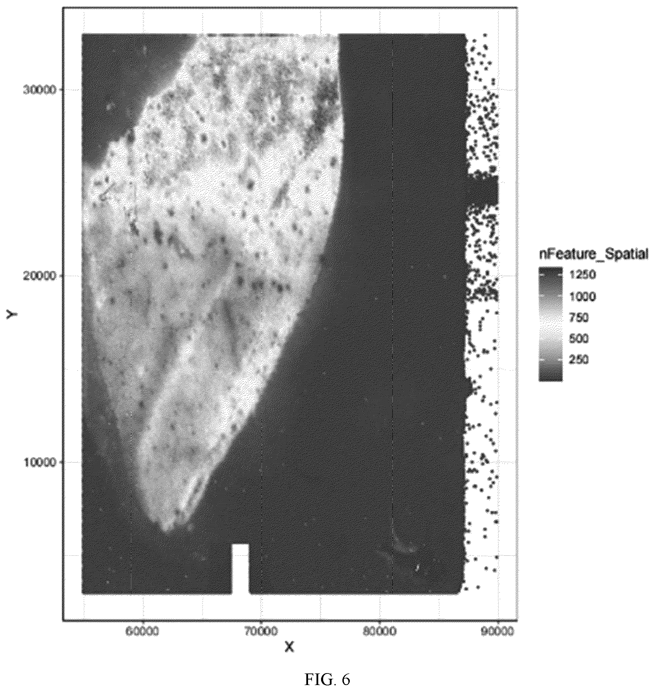

The spatial transcriptome profile of the mouse liver is constructed in accordance with the sequencing results, as shown in FIGS. 5 and 6, wherein FIG. 5 shows the spatial distribution heatmap of mRNA capture, while FIG. 6 shows the spatial distribution heatmap of the captured gene counts.

### EMBODIMENT 3

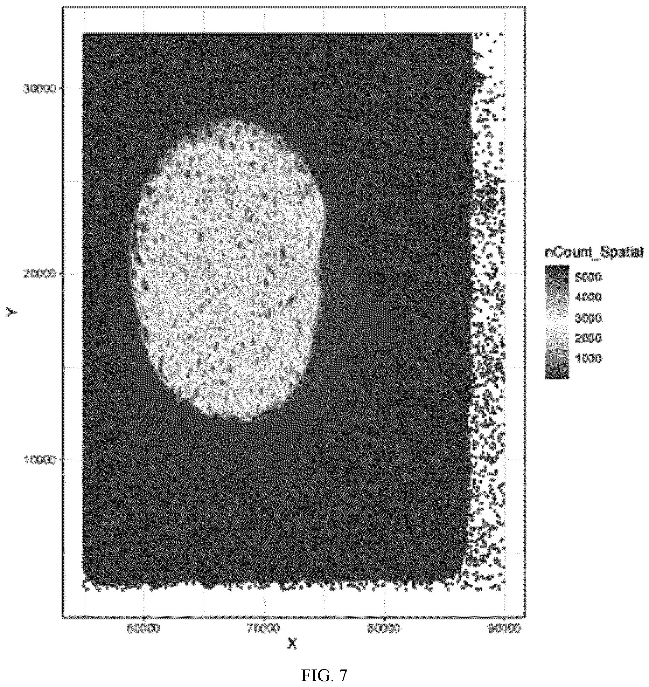

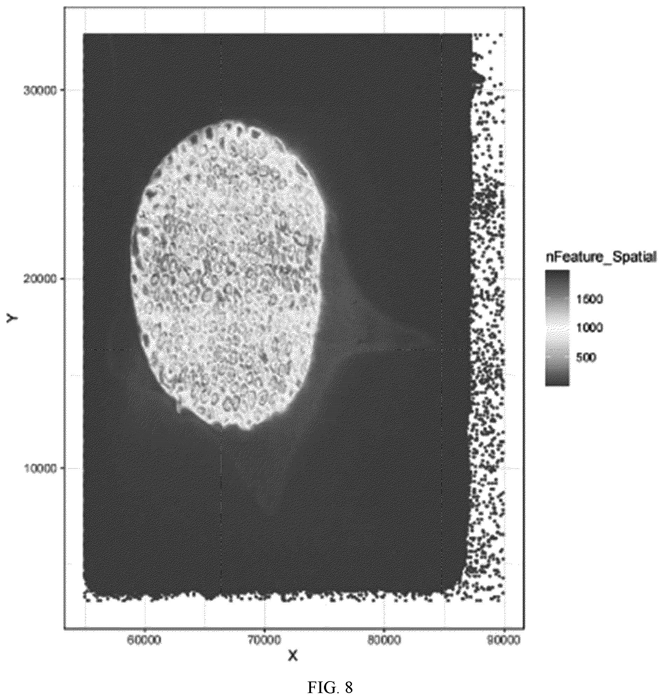

A mouse testicle tissue is cut into tissue sections with an appropriate size using a cryostat, which are attached to the mRNA capture chip obtained in EMBODIMENT 1. Through a tissue processing procedure similar to that of EMBODIMENT 2, the transcriptome of the mouse testicle tissue is library constructed and sequenced. The spatial transcriptome profile of the mouse testicle is constructed in accordance with the sequencing results, as shown in FIGS. 7 and 8, wherein FIG. 7 shows the spatial distribution heatmap of mRNA capture, while FIG. 8 shows the spatial distribution heatmap of the captured gene counts.

### EMBODIMENT 4

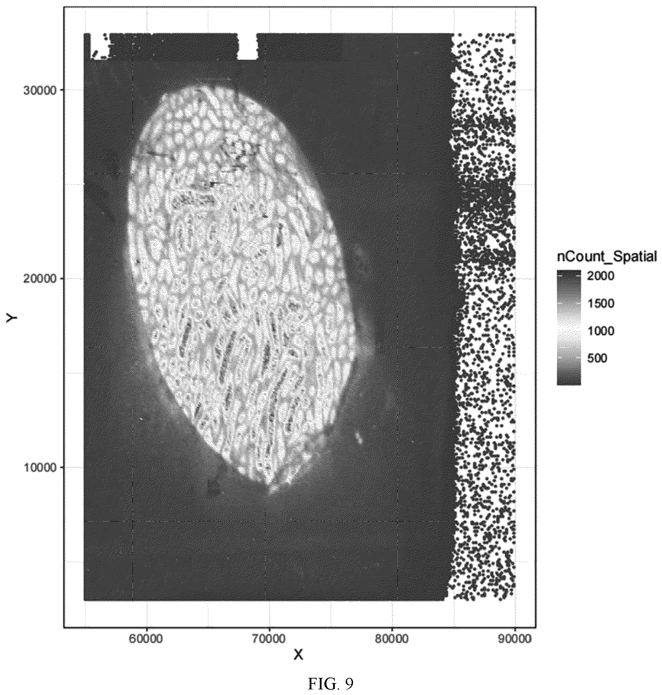

This embodiment provides a detecting method, which is different from EMBODIMENT 3 in that the poly-T sequence of the capture segment lacks locked nucleic acid modifications and consists entirely of natural nucleotides. The spatial distribution heatmap of captured mRNA molecules is shown in FIG. 9. In comparison with FIG. 7 of EMBODIMENT 3, the two embodiments have a significant difference regarding the capture counts, which indicates that the introduction of the locked nucleic acid modifications in the poly-T sequence of the capture segment can effectively enhance the mRNA capture efficiency.

### COMPARATIVE EXAMPLE 1

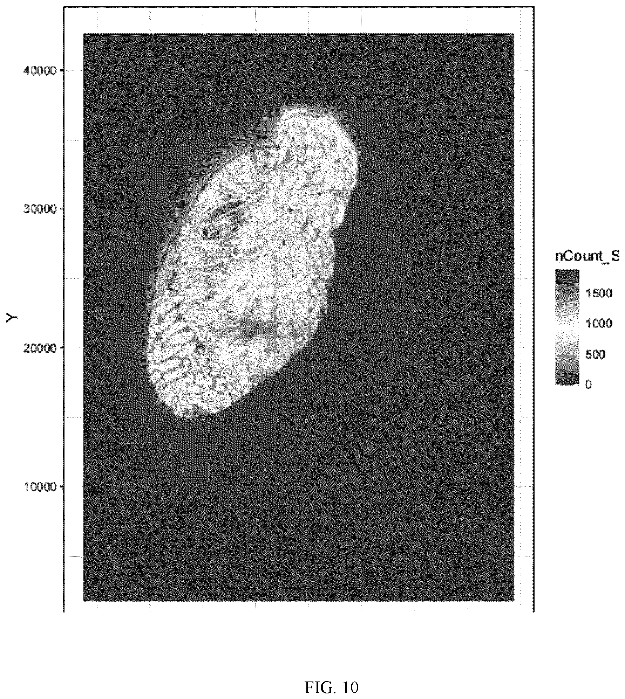

This comparative example provides a detecting method, which is different from EMBODIMENT 3 in that, during probe generation, the second amplification step is omitted. The spatial distribution heatmap of the captured mRNA molecules is shown in FIG. 10. In comparison with FIG. 7 of EMBODIMENT 3, the captured counts between the two methods are significantly different. This indicates that the re-amplification enables the chip to capture more mRNA molecules and results in a higher capture efficiency.

### COMPARATIVE EXAMPLE 2

A mouse brain tissue is cut into tissue sections with an appropriate size using a cryostat. The spatial transcriptome profile of the mouse brain tissue is constructed by using a Visium slide which is operated by following its workflow.

In the meantime, a similar tissue section is attached to the mRNA capture chip obtained in accordance with EMBODIMENT 1. The spatial transcriptome of the mouse brain tissue is conducted following an experimental process similar to EMBODIMENT 2.

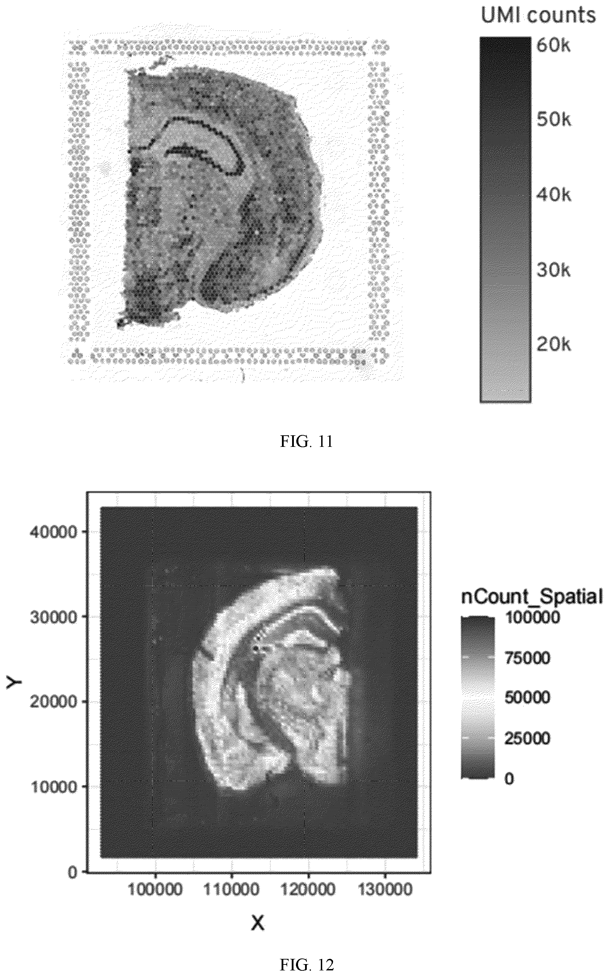

The results are shown in FIGS. 11 and 12. FIG. 11 shows the heatmap of the molecule counts of the mouse brain tissue captured using the Visium slide, while FIG. 12 shows the heatmap of the captured molecule counts of the mouse brain tissue by the present invention. As can be seen from the heatmaps of the captured mRNA molecules, the quantity of the mRNA molecules captured per unit area using the chip and the corresponding method of the present application is significantly greater than that using the Visium slide.

Although embodiments of the present application are described above in detail, the present invention should not be limited to the above embodiments, and various changes can be made without departing from the purpose of the present invention by those of ordinary skill in the art. Furthermore, the embodiments of the present invention and the features in the embodiments may be combined with each other wherever they do not conflict.

## Claims

1. A method for generating probes on a carrier, **characterized in that** the method comprises the following steps:
hybridizing a probe template with oligonucleotides immobilized on the carrier, extending the oligonucleotides to obtain probes having different spatial barcode segments;
performing first amplification to the probes to form probe clusters;
sequencing the spatial barcode segments of the probes;
performing amplification to the probe clusters; and
forming, on the probes, capture segments for capturing a target substance.

2. The method in accordance with claim 1, **characterized in that** performing amplification to the probe clusters comprises performing re-amplification to the probe clusters such that the probe clusters having different spatial barcode segments are continuously distributed on the carrier.

3. The method in accordance with claim 1, **characterized in that** the capturing segments are formed on the probes through ligase.

4. The method in accordance with claim 3, **characterized in that** the ligase comprises T4 ligase.

5. The method in accordance with claim 1, **characterized in that** the capture segments each comprise a plurality of continuous T bases.

6. The method in accordance with claim 5, **characterized in that** the number of the continuous T bases is 10-50.

7. The method in accordance with claim 5 or 6, **characterized in that** at least one or at least two of the T bases are modified with locked nucleic acid.

8. A carrier, **characterized in that** the carrier is formed with probes by using the method of any one of claims 1 to 7.

9. The carrier in accordance with claim 8, **characterized in that** the carrier is a solid-phase carrier; and/or, the solid-phase carrier comprises a flow cell.

10. A method for detecting a target substance in a sample, **characterized in that** the method comprises the following steps:
generating probes on a carrier in accordance with the method of any one of claims 1 to 7 or providing a carrier in accordance with claim 8; and
making the sample contact the carrier to enable the probes to specifically bind to the target substance through the capture segments.

11. The method in accordance with claim 10, **characterized by**:
extending the probes to obtain nucleic acid molecules containing the spatial barcode segments and the target substance; and
determining a location of the target substance in accordance with the spatial barcode segments of the nucleic acid molecules.

12. The method in accordance with claim 10, **characterized in that** the target substance is mRNA, and extending the probes to obtain nucleic acid molecules containing the spatial barcode segments and the target substance comprises: extending the probes by reverse transcription to obtain nucleic acid molecules containing the spatial barcode segments and target substance cDNA.

13. The method in accordance with claim 10, **characterized in that** determining the location of the target substance in accordance with the spatial barcode segments of the nucleic acid molecules comprises:
separating the nucleic acid molecules from the carrier, sequencing the nucleic acid molecules, and determining the location of the target substance in accordance with the sequence of the spatial barcode segments.

14. The method in accordance with claim 10, **characterized in that** the sample is a tissue section.

15. The method in accordance with claim 10, **characterized in that** making the sample contact the carrier to enable the probes to specifically bind to the target substance through the capture segments comprises: making the sample contact the carrier, and permeabilizing the sample, to enable the probes to specifically bind to the target substance in the sample through the capture segments.
